# EUROPEAN PATENT APPLICATION

(11) **EP 1 104 753 A1**
(43) Date of publication of application: **06.06.2001**
(21) Application number: 99204107.9
(22) Date of filing: 03.12.1999
(51) Int. Cl.: C07D 201/12, C08J 11/14

(54) **Process for recovering monomeric units of a nylon from whole carpet**

(71) Applicant: DSM N.V., 6411 TE Heerlen (NL)
(72) Inventor: Courage, Antonius Johannes Franciscus Maria, 6129 PP Stein (NL); Houben, Marco Johan Arnold, 5913 RM Venlo (NL); Mertens, Mathieu Hubertus Marie, 6136 BT Sittard (NL); Raets, Leonardus Joseph Gerardus, 6181 BP Elsloo (NL)
(74) Representative: Kleiborn, Paul Erik

(57) **Abstract**

The invention relates to a process for recovering monomeric units of a nylon from whole carpet comprised of fibres comprised of said nylon and a backing comprised of non-nylon components, which fibres are bound to the backing, the whole carpet containing between 15 and 35 wt.% of said nylon, which process comprises the steps of
a) mechanically separating the whole carpet into a beneficiated carpet mixture which contains between 35 and 55 wt.% of said nylon and into a depleted carpet mixture
b) exposing the beneficiated carpet mixture to conditions at which depolymerization of said nylon is effected.

## Description

The invention relates to a process for recovering monomeric units of a nylon from whole carpet.

It is generally known that monomeric units of a nylon can be recovered from whole carpet. US-A-5,681,952 describes a process in which a feed stock composed substantially of nylon-6 whole carpet can be subjected to conditions at which nylon-6 is depolymerized with sufficient yield of caprolactam. It is described that whole carpet typically contains 20 to 55 wt.% face fibre, nylon-6 often being used for the face fibre.

As a significant amount of carpets, in particular carpets for the European market, contain between 15 to 35 wt.% nylon, it is desirable that efficient processes are available for recovering monomeric units of a nylon from such carpets which contain between 15 to 35 wt.% nylon.

The invention comprises a process for recovering monomeric units of a nylon from whole carpet comprised of fibres comprised of said nylon and a backing comprised of non-nylon components, which fibres are bound to the backing, the whole carpet containing between 15 and 35 wt.% of said nylon, which process comprises the steps of
a) mechanically separating the whole carpet into a beneficiated carpet mixture which contains between 35 and 55 wt.% of said nylon and into a depleted carpet mixture
b) exposing the beneficiated carpet mixture to conditions at which depolymerization of said nylon is effected.

We have found that if the nylon content of the beneficiated carpet is below 35 wt% the processability of the beneficiated carpet during step b is decreased. We have also found that if step a) is carried out in such a way that the nylon content of the beneficiated carpet mixture is above 55 wt%, the yield of nylon and the yield of monomeric units is unsatisfactory, unless the energy which is used for step a) is increased. Preferably, the beneficiated carpet mixture contains between 38 and 50 wt.% of said nylon. The yield of nylon is defined as the mass of nylon in the beneficiated carpet mixture which is recovered from a given mass of whole carpet divided by the mass of nylon which is present in said mass of whole carpet. The yield of the monomeric units is defined as the number of monomeric units which are recovered from a given mass of whole carpet per number of monomeric units which are present in said mass of whole carpet.

Whole carpet denotes carpet of which the fibers are still bound to the backing. Accordingly, whole carpet includes, for example, carpet which has not been subjected to any mechanical separation as well as pieces or strips of carpet of which the fibres are still bound to the backing. "Fibre" denotes an elongated body, the length dimension of which is much greater than the transverse dimensions of width and thickness. Nylon may, for instance, include nylon-6, nylon-66, and nylon-46. The nylon content denotes the content of the nylon of which the monomeric units are recovered. Thus, if, for instance, caprolactam is recovered from whole carpet comprised of fibres of nylon-6, the nylon content denotes the nylon-6 content. The nylon content of a mixture is defined as the weight of the nylon of which the monomeric units are recovered in said mixture divided by the total weight of said mixture. "Backing" denotes a sheet-shaped body to which the fibres are bound, the length and width dimensions of which are much greater than the transverse dimension of thickness. The backing comprises one or more non-nylon components, such as for instance polypropylene, calcium carbonate, styrene butadiene rubber, or jute. The beneficiated carpet mixture may comprise any fraction, or combination of fractions which may be obtained by mechanical separation steps, provided that the nylon content of the beneficiated carpet mixture is between 35 and 55 wt.% nylon. The depleted carpet mixture denotes all materials which are obtained in step a) except for the beneficiated carpet mixture.

Step a) may be carried out using any combination of mechanical steps which are known in the art, such as for instance shredding, tearing, hammering, screening, granulation and air separation steps. Preferably, step a) comprises the steps of
a1) pulverizing and loosening the backing from the fibres while leaving the fibres essentially intact to obtain a carpet mixture comprised of backing particles and fibres
a2) sieving the carpet mixture to obtain a first rich carpet mixture having a higher nylon content than the carpet mixture and a first poor carpet mixture having a lower nylon content than the carpet mixture
a3) incorporating the first rich carpet mixture in the beneficiated carpet mixture.

Incorporating the first rich carpet mixture in the beneficiated carpet mixture is understood to be including the first rich carpet mixture in the beneficiated carpet mixture without subjecting the first rich carpet mixture to additional separation steps in which the nylon content of the first rich carpet mixture would substantially be increased. This embodiment has the advantage that at least a part of the beneficiated carpet mixture is obtained using only a few separation steps. Not subjecting the first rich carpet mixture to additional mechanical separation steps, such as for instance granulation, gives the advantage that energy is saved. An example of a step in which the nylon content of the first rich carpet mixture is not considered to be substantially increased, is an optional separation of metal from the first rich carpet mixture.
Step a1) may be carried out by any known means. In an embodiment of the invention step a1) involves shredding the whole carpet into strips after which said strips are impacted against an aperture anvil plate with hammer elements. This method has been described in US-A-5,535,945. Preferably step al) is carried out using a shredder, the shredder being provided with knives, the knives being sufficiently blunt to effect tearing of the backing from the whole carpet and pulverizing of the backing.

It is noted that in case of the usual operation of a shredder, which has for instance been described in US-A-5,535,945, the knives are sufficiently sharp to cut the whole carpet into strips. In that case the fibres are not substantially loosened from the backing, and no substantial pulverizing of the backing is achieved, so that an additional hammering step is required to pulverize the backing. If the knives of the shredder are sufficiently blunt, one single mechanical separation step is sufficient to obtain the carpet mixture from whole carpet even if this has not been cut into strips. In this embodiment the shredder preferably comprises a screen and a rotor with the knives, and the screen preferably surrounds the rotor, leaving space between the screen and the rotor, so that whole carpet may be introduced into said space. Preferably the diameter of the openings in the screen is between 5 and 60 mm, more preferably between 10 and 40 mm. An increasing diameter of the openings has generally the effect that the backing is pulverized to a lesser extent. A decreasing diameter of the openings has generally the effect that the amount of carpet which may be treated per unit of time, is decreased. The shredder is not limited to a specific type. A very suitable shredder is the VAZ 300/250 UNF shredder which is manufactured by Vecoplan Maschinefabrik GMBH.

Step a2) usually involves supplying the carpet mixture to one or more screens with openings. Usually, the backing particles are transported more easily through the openings of the screen than the fibres. Thus a mixture consisting of fibres and backing particles which have not been transported through the openings will generally have a higher nylon content than a mixture which consists of fibres and backing particles which have been transported through the openings. The first rich carpet mixture may be any mixture of fibres and backing particles which has been obtained by sieving of the carpet mixture provided that said mixture of fibres and backing particles has a higher nylon content than the carpet mixture. Thus, if only one screen is used, the first rich carpet mixture generally comprises the backing particles and the fibres which have not been transported through the openings of the screen. It is also possible that sieving is carried out more than one time, i.e. fibres and backing particles which have been transported through the openings of a first screen may be sieved again, for instance by using a second screen of having openings which sizes are different from those in the first screen. In that case, fibres and backing particles which have not been transported through the openings of the first screen as well as fibres and backing particles which have been transported through the openings of the first screen, but not through the openings of the second screen, may together form the first rich carpet mixture, provided that the nylon content of the first rich carpet mixture is higher than that of the carpet mixture.

Step a2) may be carried out by any known means, for instance by using shaking screens, rotating drum screens, optionally in combination with gas flushing. Preferably, step a2) is carried out using an elastic flapping screen. This has the advantage that blocking of the openings of the screen by clusters of fibres is minimized. An elastic flapping screen is understood to be a screen which has been made of an elastic material, for instance rubber, and to which energy may be supplied during the sieving, for instance by stretching and unstreching the screen in an alternating way. A very suitable sieving apparatus is a bivi-TEC double deck screening machine, designed by Binder & Co. AG, Germany, and manufactured by Jöst, Munster, Germany. The dimensions of the openings are not essential for the invention. Usually, the radius of the circumscribed circle of the openings is between 1 and 100 mm, preferably between 2 and 50 mm.

The nylon content of the first rich carpet mixture may be varied in various ways. If only one screen is used, the nylon content of the first rich carpet mixture may for instance be increased by increasing the residence time of the carpet mixture on the screen. If the carpet mixture is sieved into more than two fractions, the nylon content of the first rich carpet mixture may be varied by making various combinations of fractions. We have found that the sieving yield, i.e. the total mass of the nylon in the first rich carpet mixture divided by the total mass of the nylon in carpet mixture is decreased with increasing nylon content of the first rich carpet mixture. Preferably, step a2) is carried out in such a way that the first rich carpet mixture contains between 35 and 55 wt.% of said nylon, more preferably between 38 and 50 wt.%. This gives the advantage that a significant part of the beneficiated carpet mixture is obtained in only a few mechanical steps.

In a preferred embodiment, step a) further comprises the steps of
a4) mechanically separating the first poor carpet mixture into a second rich carpet mixture having a higher nylon content than the first poor carpet mixture and into a second poor carpet mixture
a5) incorporating the second rich carpet mixture in the beneficiated carpet mixture

This embodiment gives the advantage that the yield of nylon and the yield of monomers of said nylon is increased. Step a4) may be carried out using any known technique, for instance by shredding, hammering, screening, granulation, air separation, or combinations thereof.

The process according to the invention is not limited to recovering of monomeric units of a specific nylon. Preferably, the nylon is nylon-6.

Step b) is not limited to a specific method. If the process according to the invention is used for recovering caprolactam from whole carpet comprised of nylon-6, the depolymerization may for instance be effected using the methods taught by WO-A-9618613 or US-A-5,169,870. If the process according to the invention is used for recovering caprolactam from whole carpet comprised of nylon-6, step b preferably comprises the step bl) contacting the beneficiated carpet mixture with superheated steam at a temperature of about 250 °C to about 400 °C and at a pressure within the range of about 1 atm. to about 100 atm. and substantially less than the saturated vapor pressure of water at said temperature wherein a caprolactam-containing vapor stream is formed. This method has been described in US-5,681,952.

The invention will now be elucidated with reference to the following examples, without however being limited thereto.

### Examples

- a VAZ 300/250 UNF shredder which is manufactured by Vecoplan Maschinefabrik GMBH was used which was equipped with a screen of which the openings had a diameter of 30 mm and with a rotor to which 56 blunt knives and no sharp knives were attached.
- a bivi-TEC double decks screening machine, designed by Binder & Co. AG, Germany, and manufactured by Jöst, Munster, Germany were used. The screening machine contained two sieve decks, one sieve deck mounted below the other. The decks could each be fitted with 14 sieve plates with a width of 0.4 metre and a length of 1.5 m. Each of these sieve plates could have one of the following opening sizes: 2x8 mm, 3x10 mm, 10x24 mm, 15x25 mm, 22x30 mm, 26x32 mm or 28x35 mm.

### Example I

Carpets which had not been shredded into strips (average composition 27.3 wt.% nylon-6, 4.6 wt.% polypropylene, 39.0 wt.% CaCO₃ and 27.9 wt.% styrene butadien rubber) were fed to the shredder at an average rate of 2500 kg/hour. The shredder was operated at 150 rpm. A carpet mixture comprised of backing particles and nylon fibres was recovered from the shredder. The carpet mixture was distributed over the upper deck of the Jöst sieve. The upper deck was fitted with 7 sieve plates with openings of 26x32 mm and 7 sieve plates with openings of 28x35 mm, placed in random order. The lower deck was fitted with 7 sieve plates with openings of 2x8 mm and 7 sieve plates with openings of 3x10 mm, placed in random order. Three streams of material were obtained which were continuously recovered. At some point in time 480 kg material with a nylon content of 47.1 wt.% had been recovered, which had not been transported through the openings of the upper deck, 2710 kg material with a nylon content of 33.7 wt.%, which had been transported throught the openings of the upper deck, but not through the openings of the lower deck, and 1330 kg material with a nylon content of 10.7 wt.% which had passed through the openings of both decks.

A first rich carpet mixture consisting of the 480 kg material with a nylon content of 47.1 wt.% corresponds to a nylon yield of 18 wt.%.

### Example II

A first rich carpet mixture consisting of the 480 kg material with a nylon content of 47.1 wt.% as obtained in example I and the 2710 kg material with a nylon content of 33.7 wt.% as obtained in example I has an average nylon content of 36 wt.%. The nylon yield is 89 wt.%.

### Example III

Carpets which had not been shredded into strips (average composition 28.5 wt.% nylon, 4.6 wt.% polypropylene, 36.6 wt.% CaCO₃ and 26.9 wt.% styrene butadien rubber) were fed to the shredder at an average rate of 2500 kg/hour. The shredder was operated at 150 rpm. A carpet mixture comprised of backing particles and nylon fibres was recovered from the shredder. The carpet mixture was distributed over the upper deck of Jöst sieve. Starting from the feed position of the sieve, the upper deck was fitted with 7 sieve plates with openings of 15x25 mm, followed by 7 sieve plates with openings of 28x35 mm. Starting from the feed position of the sieve, the lower deck was fitted with 4 sieve plates with openings of 2x8 mm, followed by 10 sieve plates with openings of 10x24 mm. Three streams of material were obtained which were continuously recovered. At some point in time 510 kg material with a nylon content of 41.3 wt.% had been recovered, which had not been transported through the openings of the upper deck, 180 kg material with a nylon content of 34.3 wt.%, which had been transported throught the openings of the upper deck, but not through the openings of the lower deck, and 810 kg material with a nylon content of 16.3 wt.% which had passed through the openings of both decks.

A first rich carpet mixture consisting of the 510 kg material with a nylon content of 41.3 wt.% corresponds to a nylon yield of 52 wt.%.

### Example IV

A first rich carpet mixture consisting of the 510 kg material with a nylon content of 41.3 wt.% as obtained in example III and the 180 kg material with a nylon content of 34.3 wt.% as obtained in example III, has an average nylon content of 39 wt.%. The nylon yield is 67 wt.%.

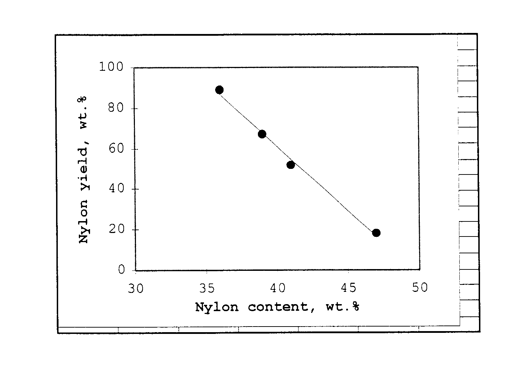

Fig. 1 shows the nylon yield as a funtion of the nylon content for the first rich carpet mixtures as obtained in examples I to IV. The nylon yield has been plotted as a function of the nylon content in the rich carpet mixture. It can clearly be seen that the nylon yield decreases with increasing nylon content in the rich carpet mixture. The first rich carpet mixtures obtained are suitable to be exposed to conditions at which depolymerization of the nylon is effected.

## Claims

1. Process for recovering monomeric units of a nylon from whole carpet comprised of fibres comprised of said nylon and a backing comprised of non-nylon components, which fibres are bound to the backing, the whole carpet containing between 15 and 35 wt.% of said nylon, which process comprises the steps of
a) mechanically separating the whole carpet into a beneficiated carpet mixture which contains between 35 and 55 wt.% of said nylon and into a depleted carpet mixture
b) exposing the beneficiated carpet mixture to conditions at which depolymerization of said nylon is effected.

2. Process according to claim 1, characterized in that the beneficiated carpet mixture contains between 38 and 50 wt.% of said nylon.

3. Process according to claim 1 or claim 2,
characterized in that step a) comprises the steps of
a1) pulverizing and loosening the backing from the fibres while leaving the fibres essentially intact to obtain a carpet mixture comprised of backing particles and fibres
a2) sieving the carpet mixture to obtain a first rich carpet mixture having a higher nylon content than the carpet mixture and a first poor carpet mixture having a lower nylon content than the carpet mixture
a3) incorporating the first rich carpet mixture in the beneficiated carpet mixture.

4. Process according to claim 3, characterized in that step a1) is carried out using a shredder, that the shredder is provided with knives, the knives being sufficiently blunt to effect tearing of the backing from the whole carpet and pulverizing of the backing.

5. Process according to claim 3 or claim 4,
characterized in that step a2) is carried out using an elastic flapping screen.

6. Process according to any one of claims 3 to 5, characterized in that step a2) is carried out in such a way that the first rich carpet mixture contains between 35 and 55 wt.% of said nylon.

7. Process according to any one of claims 3 to 6, characterized in that step a) further comprises the steps of
a4) mechanically separating the first poor carpet mixture into a second rich carpet mixture having a higher nylon content than the first poor carpet mixture and into a second poor carpet mixture
a5) incorporating the second rich carpet mixture in the beneficiated carpet mixture.

8. Process according to any one of claims 1 to 7, characterized in that said monomeric units are caprolactam and that said nylon is nylon-6.

9. Process according to claim 8, characterized in that step b) comprises the step
b1) contacting the beneficiated carpet mixture with superheated steam at a temperature of about 250 °C to about 400 °C and at a pressure within the range of about 1 atm. to about 100 atm. and substantially less than the saturated vapor pressure of water at said temperature wherein a caprolactam-containing vapor stream is formed.
